# EUROPEAN PATENT APPLICATION

(11) **EP 1 739 182 A1**
(43) Date of publication of application: **03.01.2007**
(21) Application number: 05719892.1
(22) Date of filing: 03.03.2005
(51) Int. Cl.: C12P 7/62, C12N 15/09

(54) **PROCESS FOR PRODUCING POLYHYDROXYALKANOATE**

(30) Priority: 04.03.2004 JP 2004061289
(71) Applicant: KANEKA CORPORATION, Osaka-shi, Osaka 530-8288 (JP); The Procter and Gamble Company, Cincinnati, Ohio 45202 (US)
(72) Inventor: KINOSHITA, Koichi, Kakogawa-shi, Hyogo 675-0057 (JP); YANAGIDA, Yoshifumi, 1-701, Saususukuea, Akashi-shi, Hyogo 674-0068 (JP); OSAKADA, Fumio, Okayama-shi, Okayama 700-0063 (JP); UEDA, Yasuyoshi, Himeji-shi, Hyogo 671-1227 (JP); NARASIMHAN, Karunakaran, West Chester, Ohio 45069 (US); CEARLEY, Angella, Christine, Hamilton, Ohio 45015 (US); YEE, Kenneth, Cincinnati, Ohio 45212 (US); NODA, Isao, Fairfield, Ohio 45014 (US)
(74) Representative: Vossius & Partner
(86) International application number: PCT/JP2005/003588
(87) International publication number: WO 2005/085460

(57) **Abstract**

The present invention provides a method for obtaining a biodegradable polyhydroxyalkanoate by a solvent extraction method without causing a significant molecular weight decrease.

The present invention relates to a method for producing a polyhydroxyalkanoate which comprises extracting a polyhydroxyalkanoate from a polyhydroxyalkanoate-containing biomass having the water content of 5% by weight or less using an extraction solvent, crystallizing, and recovering the resultant.

## Description

### TECHNICAL FIELD

The present invention relates to a method for producing a polyhydroxyalkanoate.

### BACKGROUD ART

A polyhydroxyalkanoate (hereinafter referred to' briefly as "PHA") is a biodegradable and thermoplastic polyester which is synthesized and accumulated as an energy storage substance in cells of a variety of microorganisms. A PHA, which is produced by microorganisms using natural organic acids or oils as carbon sources, is completely biodegraded by a microorganism in soil or water to be taken up in the carbon cycle of the natural world. Therefore, a PHA can be said to be an environment-conscious plastic material which hardly causes adverse effects for ecological system. In these years, a synthetic plastic came into a serious social problem in view of environment pollution, waste disposal and oil resource, thus a PHA has attracted attention as an eco-friendly green plastic and its practical applications are longed for.

As a PHA-producing biomass, there is a microorganism innately producing a PHA, or a transformant obtainable by recombinationg a PHA synthase gene into a microorganism or a plant. In both cases, since a PHA is accumulated in the biomasses, the PHA is to be produced by recovering the PHA-containing biomass, and separating and purifying the PHA from the biomass.

As regarding the separation and purification of a PHA from a biomass, a method is known as the most convenient which comprises extracting a PHA using a PHA-soluble solvent, crystallizing the resultant using a poor solvent, and recovering the PHA as a crystal. For example, there is disclosed a method comprising drying a biomass in which a PHA is accumulated, extracting the PHA using a halogen-containing organic solvent such as chloroform and methylene chloride, and then mixing the extract with a poor solvent such as methanol and hexane to precipitate and recover the PHA (see Japanese Kokai Publication Sho-59-205992 and United States Patent No.4324907). However, since these halogen-containing organic solvents are in connection with the environmental regulation, the usage restriction is strict, thus they cannot substantially be used on a commercial scale production. Accordingly, a study has been made for an extraction using a halogen-free organic solvent without using a halogen-containing organic solvent.

However, since a PHA has quite a low solubility in halogen-free solvents (United States Patent No.6043063), a huge amount of solvent is required on a commercial scale production. Thus, a study has been made directed to reduce the amount of solvent as much as possible by extracting at high temperature to increase the solubility (United States Patent No.6043063, United States Patent No.5894062, and United States Patent No.6087471). However, regardless of the solvent species, a heating extraction at high temperature tends to significantly decrease the PHA molecular weight with the extraction time (United States Patent No.4101533).

Although many of the prior art documents do not refer to the molecular weight decrease at the time of extraction, it is assumed that a problem has not been caused since the extraction is carried out in quite a short time (United States Patent No.6043063 and United States Patent No.5894062), or a purified polymer is used for extraction (United States Patent No.6043063). However, the present inventors experienced that when, for example, an extraction was carried out from a cell as biomass, the molecular weight of a PHA significantly decreased after the extraction for several or more hours, and the PHA becomes disqualified as a product. When the commercial scale mass production is carried out, it is sufficiently presumable that a polymer is exposed to high temperature over several hours from the start of extraction through removal of residues other than PHA to crystallization. During this time, it is sufficiently possible that the molecular weight of the polymer decreases to an extent that a processing becomes impossible.

Within cells, there are many impurities such as a polymer degrading enzyme, cytoplasmic membrane, cell wall component, lipid, nucleic acid, and protein. In the heating extraction, it is considered that the molecular weight of a polymer decreases by a synergistic interaction between these impurities and a solvent. In United States Patent No.5821299, a PHA is extracted using a solvent after lipid-soluble impurities are washed and removed with a solvent. By this operation, the molecular weight decrease may possibly be suppressed to some extent. However, this method is disadvantageous in that operations are complicated on a commercial scale, a lot of solvents are separately required, and equipment costs become high.

Since a PHA is low in solubility, it is desirable to carry out an extraction at a concentration as high as possible. But higher the concentration, the more a sequence of operations including a residue removal after the extraction tends to be complicated and require a long time, thus the significant molecular weight decrease is concerned. However, there has still not been found a technology for suppressing the molecular weight decrease in a sequence of operations assuming actual equipment. Therefore, the actual state is that the solvent extraction method which is considered to be substantially convenient has not been put into practical use.

### SUMMARY OF THE INVENTION

Accordingly, the subject of the present invention is to provide a commercially preferable method for producing a polyhydroxyalkanoate having high processability while suppressing the molecular weight decrease of the polyhydroxyalkanoate when extracting the polyhydroxyalkanoate from a polyhydroxyalkanoate-containing biomass using a solvent.

The present inventors have eagerly investigated on the above-mentioned subject. As a result, they have surprisingly found that, for the first time, the significant molecular weight decrease of a polyhydroxyalkanoate at the time of a solvent extraction can be suppressed by controlling the water content in a biomass to 5% by weight or less. Thereby, they completed the present invention. By this procedure, it becomes possible to extract a high molecular weight polyhydroxyalkanoate directly from a biomass without removing impurities by a solvent washing before extracting the polyhydroxyalkanoate.

That is, the present invention relates to a method for producing a polyhydroxyalkanoate from a polyhydroxyalkanoate-containing biomass which comprises extracting a polyhydroxyalkanoate from a polyhydroxyalkanoate-containing biomass whose water content has been controlled to 5% by weight or less using an extraction solvent, crystallizing, and recovering the resultant.

### DETAILED DESCRIPTION OF THE INVENTION

In the following, the present invention is described in detail.

According to the present invention, the water content in a polyhydroxyalkanoate (PHA)-containing biomass is 5% by weight or less. It is preferably 3% by weight or less, and still more preferably 2% by weight or less in view of obtaining more preferable suppressing effect of the PHA molecular weight decrease. Preferred lower limit is 0% by weight, and more preferred lower limit is 1% by weight.

The water content in a biomass is preferably measured by, for example, an infrared water balance manufactured by such as Kett Electric Laboratory or Sanko Electric Laboratory Co., Ltd. since the measurement can be carried out simply and quickly. But equipment is not restricted to these. In the present invention, the infrared water balance manufactured by Kett Electric Laboratory is used, and the measurement is carried out by the following method. That is, 1 to 2 g of a sample is prepared in said balance, and dried at 130°C for approximately 15 minutes until the weighing value becomes equilibrium. The same sample is weighed 3 times and the average of the three values is determined as the water content.

For controlling the water content in the biomass used for the present invention to 5% by weight or less, a method is preferred which comprises drying by heating, or a method which comprises reducing the water content of a biomass by concentration and/or azeotropic dehydration under the coexistence of a solvent. However, these methods are not any restriction.

As equipment for drying by heating which can be used for the purpose of the present invention, for example, a spray drier, vacuum incubation drier, drum heater, high-temperature heating furnace, ceramic heater, silicone rubber heater, high frequency continuous heating equipment, far-infrared radiation heater, microwave heating equipment, etc. can be suitably used. However, equipment is not restricted to these, and those with which the desired water content can be attained may be used. Surely, it is also possible to combinedly use these equipment to attain the desired water content.

In the case of drying by heating, a PHA-containing biomass is preferably exposed to heat environment of 40°C or higher, and more preferably 50°C or higher. The above drying by heating is preferably carried out within the temperature and time ranges that the molecular weight decrease by heating does not occur. Preferable upper limit of the temperature is 100°C, and more preferable upper limit is 90°C. Furthermore, the above drying by heating is preferably carried out under reduced pressure.

When the water content in a biomass is decreased by concentration and/or azeotropic dehydration under the coexistence of a solvent, as said solvent, for example, toluene, butanol, ethyl acetate, etc. may be used. In addition, the concentration and/or azeotropic dehydration under the coexistence of a solvent may be carried out either at 40°C or higher, or below 40°C.

In the present invention, in the process for controlling the water content in a biomass to 5% by weight or less, when the biomass is a cell, the cultured broth can be used as it is. Alternatively, a wet cell recovered by a method such as centrifugation or membrane separation can also be used.

In the present invention, by controlling the water content in a biomass to 5% by weight or less, the molecular weight decrease of a PHA in the solvent extraction at high temperature can be suppressed. Therefore, an extraction can be completed in one step without removing impurities other than the polyhydroxyalkanoate before the extraction.

In the production method of the present invention, a polyhydroxyalkanoate is extracted by adding an extraction solvent to a PHA-containing biomass having the water content of 5% by weight or less. The weight ratio of a PHA in the solvent extraction is not particularly restricted, but it is preferably 1 to 20% by weight relative to the total amount of the PHA and extraction solvent. More preferable lower limit is 2% by weight, and more preferable upper limit is 15% by weight. Still more preferable lower limit is 3% by weight, and still more preferable upper limit is 10% by weight in view of reducing the amount to be used of a solvent as much as possible, and carrying out the extraction at high efficiency.

The temperature for extracting a PHA is preferably higher than 50°C, more preferably higher than 55°C, and still more preferably higher than 60°C. However, in order to prevent decomposition of a PHA as much as possible, it is preferable that the extraction temperature does not substantially exceed 100°C over 3 hours. Furthermore, when an extraction solvent with a low boiling point is used, it is preferable to carry out extraction under a pressurized condition at a temperature under the boiling point.

Duration for extracting a PHA is not particularly restricted, but preferably 10 to 150 minutes, and more preferably 30 to 120 minutes in view of obtaining preferable extraction efficiency and preventing the decomposition of a PHA.

In the production method of the present invention, it is preferable to separate a PHA and insoluble biomass after the extraction. The separation of a PHA and insoluble biomass can be carried out by the methods well-known to the person skilled in the art. In this case, it is preferable to use a closed pressurized filter, reduced pressure filter, centrifugal separator, decanter type separator, and the like.

According to the preferred embodiment of the present invention, after a PHA is extracted using an extraction solvent, the PHA solution is preferably kept warm. The temperature for the warming mentioned above is preferably 50°C or higher, more preferably 55°C or higher, and still more preferably 60°C or higher. If the temperature drops to below 50°C, a PHA begins to gelate without having fluidity, solidifies later, and becomes a state which cannot be recovered. However, to prevent decomposition of a PHA, it is preferable that the temperature for the warming mentioned above does not substantially exceed 100°C.

Moreover, the above warming is preferably continued until the subsequent crystallization is completed.

Furthermore, according to the preferred embodiment of the present invention, the crystallization of a PHA from a PHA solution is preferably carried out by gradually adding a poor solvent for crystallization to the above warmed PHA solution, and cooling the solution to below 50°C, or preferably to near room temperature while vigorously stirring to precipitate the dissolved PHA almost completely. The amount to be added of the poor solvent for crystallization is preferably such that the weight ratio of the poor solvent for crystallization relative to the total amount of the poor solvent for crystallization and extraction solvent is 10 to 70% by weight. More preferable lower limit is 20% by weight, and more preferable upper limit is 60% by weight. By this procedure, it becomes possible to obtain a PHA having fluidity, capable of being brushed away, and further having a low liquid content, which have conventionally been very difficult to obtain.

In the present invention, to be put into practical use, the PHA preferably has the weight average molecular weight determined by a gel chromatography method, in which polystyrene is set as a molecular weight standard, of 10,000 or more.

It is natural that an appropriate molecular weight varies according to various applications. However, taking the molecular weight decrease by heat in pelletization or at the subsequent processing stage into consideration, the weight average molecular weight of the PHA which is recovered after crystallization and dried is preferably 400,000 or more, and particularly preferably 500,000 or more.

The extraction solvent used in the present invention is a solvent dissolving 3% by weight or more of a PHA at its boiling point, but preferably one having a solubility of 4% by weight or more, more preferably 5% by weight or more, and particularly preferably 6% by weight or more. The extraction solvent is preferably at least one species selected from the group consisting of monohydric alcohols having 1 to 10 carbon atoms, aromatic hydrocarbons having 6 to 10 carbon atoms, ketones having 4 to 7 carbon atoms, and fatty acid alkyl esters having 5 to 8 carbon atoms.

As the monohydric alcohols having 1 to 10 carbon atoms, there may be mentioned methanol, ethanol, propanol, butanol, pentanol, hexanol, cyclohexanol, 1-methylcyclohexanol, 2-ethylhexanol, benzyl alcohol, heptanol, octanol, nonanol, decanol, isomers thereof (e.g. n-butanol, isobutanol, 2-methyl-1-butanol, 3-methyl-l-butanol, n-pentanol, 2-pentanol, 3-pentanol, 1-hexanol, 2-hexanol, 1-heptanol, 2-heptanol, 3-heptanol, 1-octanol, 2-octanol, 1-nonanol, 2-nonanol, 1-decanol, 2-decanol, etc.), and the like. As the above-mentioned monohydric alcohols having 1 to 10 carbon atoms, preferred is at least one species selected from the group consisting of butanol, pentanol, hexanol, cyclohexanol, 1-methylcyclohexanol, 2-ethylhexanol, benzyl alcohol, heptanol, octanol, nonanol, decanol, and isomers thereof.

As the aromatic hydrocarbons having 6 to 10 carbon atoms, preferred are benzene, toluene, xylene, ethyl benzene, dimethoxybenzene, trimethylbenzene, cumene, butyl benzene, cymene, and isomers thereof (e.g. 1,3-dimethoxybenzene, 1,2,3-trimethylbenzene, 1,2,4-trimethylbenzene, o-xylene, m-xylene, p-xylene, o-cymene, m-cymene, p-cymene, etc.).

As the ketones having 4 to 7 carbon atoms, preferred are methyl ethyl ketone, methyl butyl ketone, pentanon, hexanon, cyclohexanone, heptanone, and isomers thereof (e.g. methyl isobutyl ketone, methyl n-amyl ketone, 2-hexanone, 3-hexanone, 5-methyl-2-hexanone, etc.).

Moreover, as the fatty acid alkyl esters having 5 to 8 carbon atoms, preferred are propyl acetate, butyl acetate, pentyl acetate, hexyl acetate, and isomers thereof (e.g. isobutyl acetate, ethyl butyrate, isoamyl acetate, propyl propionate, butyl propionate, pentyl propionate, butyl butyrate, isobutyl isobutyrate, ethyl butyrate, ethyl valerate, methyl valerate, etc.). The extraction solvent mentioned above may be used one species or two or more.

Among these extraction solvents, particularly preferred are n-butanol, isobutanol, n-pentanol, 2-pentanol, 3-pentanol, toluene, benzene, methyl ethyl ketone, butyl acetate, butyl propionate, and ethyl acetate as the extraction solvents of the present invention in view of having high solubility of a PHA. Among these extraction solvents, aromatic hydrocarbons and ketones, i.e. toluene, benzene and methyl ethyl ketone are preferred in view of preventing the molecular weight decrease of a PHA in dissolution, but toluene is more preferred for its comparatively low cost.

The poor solvent for crystallization used in the present invention is a solvent which does not dissolve 0.5% by weight or more of a PHA at 15 to 25°C, and is preferably a solvent which does not dissolve 0.3% by weight or more of a PHA. As the poor solvent for crystallization mentioned above, preferred are aliphatic hydrocarbons having 6 to 12 carbon atoms with the boiling point of 60°C or more. For example, there may be mentioned hexane, heptane, methylcyclohexane, octane, nonane, decane, dodecane, undecane, isomers thereof (e.g. n-heptane, isoheptane, etc.), and the like. Among these poor solvents for crystallization, heptane and methylcyclohexane are preferred as the poor solvent for crystallization of the present invention. Heptane is more preferred, and n-heptane is particularly preferred as heptane.

The recovery of PHA after the crystallization is carried out by the methods well-known to the person skilled in the art such as a liquid filtration or centrifugation of a PHA solution after crystallization. The recovered PHA is preferably washed with an appropriate poor solvent. As such poor solvent to be used in the above washing, for example, solvents such as water, methanol, ethanol, acetone and hexane, or a mixture thereof with the above-mentioned extraction solvents can be used. The drying of PHA recovered is carried out by the methods well-known to the person skilled in the art such as, for example, air flush drying and vacuum drying. But the method is not restricted to these.

The PHA of the present invention is not particularly restricted as for its hydroxyalkanoate components, but specifically, there may be mentioned 3-hydroxybutyrate(3HB), 3-hydroxyvalerate(3HV), 3-hydroxypropionate, 4-hydroxybutyrate, 4-hydroxyvalerate, 5-hydroxyvalerate, 3-hydroxyhexanoate(3HH), 3-hydroxyheptanoate, 3-hydroxyoctanoate, 3-hydroxynonanoate, 3-hydroxydecanoate, etc.

The PHA of the present invention may be a homopolymer of one of these hydroxyalkanoates or a copolymer obtainable by copolymerizing at least two or more species of these. As specific examples of the PHA, there may be mentioned PHB (a homopolymer of 3HB), PHBV (a binary copolymer composed of 3HB and 3HV), PHBH (a binary copolymer composed of 3HB and 3HH, see Japanese Patent Publication No. 2777757), PHBHV (a ternary copolymer composed of 3HB, 3HV and 3HH, see Japanese Patent Publication No. 2777757), etc. The PHA of the present invention is preferably a copolymer obtainable by copolymerizing at least two species of the hydroxyalkanoates mentioned above.

Particularly among them, a copolymer comprising 3HH and at least one species of other hydroxyalkanoates as monomer components is preferable since it has degradability as a biodegradable polymer and softness, and more preferred is PHBH. In this case, the compositional ratio of monomer units constituting PHBH is not particularly restricted but ones containing 40 mol% or less of 3HH unit are preferred and ones containing 30 mol% or less of 3HH units are more preferred, and ones containing 20 mol% or less of 3HH units are particularly preferred in view of preferable crystallinity in the crystallization. In the case of PHBHV, the compositional ratio of monomer units constituting of PHBHV is not particularly restricted, but for example, ones containing 1 to 95 mol% of 3HB unit, 1 to 96 mol% of 3HV unit, and 1 to 30 mol% of 3HH unit are preferred.

The biomass to be used in the present invention is not particularly restricted provided that it is a living organism capable of accumulating a PHA in cells. However, a microorganism is preferred. For example, microorganisms belonging to the genus Alcaligenes such as Alcaligenes lipolytica and Alcaligenes latus, the genus Ralstonia such as Ralstonia eutropha, the genus Pseudomonas such as Pseudomonas oleovorance and Pseudomonas resinovorans, the genus Bacillus, the genus Azotobacter, the genus Nocardia such as Nocardia salmonicolur, the genus Aeromonas such as Aeromonas caviae, the genus Rhodospirillum such as Rhodospirillum rubrum, the genus Zoogloea such as Zoogloea ramigera, the genus Methylobacterium, the genus Paracoccus, the genus Clostridium, the genus Halobacterium, the genus Candida, the genus Yarrowia, the genus Saccharomyces and the like can accumulate a PHA in cells by controlling culture conditions.

The PHA of the present invention is preferably produced by at least one species of cell selected from the biomass group consisting of the genus Aeromonas, the genus Alcaligenes, the genus Azotobacter, the genus Bacillus, the genus Clostridium, the genus Halobacterium, the genus Nocardia, the genus Rhodospirillum, the genus Pseudomonas, the genus Ralstonia, the genus Zoogloea, the genus Candida, the genus Yarrowia, and the genus Saccharomyces.

Alternatively, the biomass used in the present invention may also be a transformant obtainable by introducing a gene group involved with a PHA synthesis of these microorganisms. In that case, the host is not particularly restricted, and there may be mentioned microorganisms such as Escherichia coli and yeast (see WO 01/88144), and further plants may be mentioned in addition to the above-mentioned microorganisms. Among these, Aeromonas caviae belonging to the genus Aeromonas and the transformed cell obtainable by introducing a PHA synthase group gene derived from said Aeromonas caviae are preferable since they have a synthesizing ability of PHBH excellent as a polymer. In particular, more preferred is Ralstonia eutropha obtainable by introducing a PHA synthase group gene of Aeromonas caviae. One example of said microorganisms is internationally deposited based on Budapest Treaty to the National Institute of Advanced Industrial Science and Technology International Patent Organism Depositary, Central 6, 1-1-1 Higashi, Tsukuba, Ibaraki, Japan under the name of Alcaligenes eutrophus AC32 (accession date: August 7, 1997, accession number: FERM BP-6038).

A method for culturing the PHA-producing microorganisms mentioned hereinabove is not particularly restricted, but for example, the method well-known to the person skilled in the art disclosed in Japanese Kokai Publication 2001-340078 can be used.

In recovering a PHA, it is naturally preferable that the PHA content in the cultured microbial cell is higher. In the application for a commercial scale production, the PHA content is preferably 50% by weight or more in a PHA-containing biomass whose water content has been controlled to 5% by weight or less. The PHA content is more preferably 60% by weight or more, and particularly preferably 70% by weight or more.

The PHA-containing biomass residues after being treated according to the present invention are preferably used as animal feed, microorganism feed, or vegetable fertilizer. Accordingly, the extraction solvent to be used in the present invention is preferably in such an amount that is permissible as feed or fertilizer. However, it is preferable to substantially remove the solvent from the biomass substances.

The PHA obtained by the production method according to the present invention may be formed into various forms, such as fibers, threads, ropes, textiles, fabrics, nonwoven fabrics, papers, films, sheets, tubes, boards, sticks, containers, bags, parts, and foamed bodies. Moreover, it may also be processed into a biaxial stretched film. The formed products may be suitably used for such fields as agriculture, fishery, forestry, gardening, medical, sanitary products, clothing, non-clothing, packaging, and others.

By the production method of the present invention, it becomes possible to produce and provide a biodegradable polyhydroxyalkanoate having preferable processability on a commercial scale.

### BEST MODE OF CARRYING OUT THE INVENTION

Hereinafter, the present invention will be described in further detail by way of examples. In each example, poly (3-hydroxybutyrate-co-3-hydroxyhexanoate) (hereinafter referred to briefly as "PHBH") was produced as a PHA. Surely, the present invention is not limited to these examples in its technical scope, and is not restricted to the production of PHBH.

In Examples, the water content is measured using the infrared water balance FD230 manufactured by Kett Electric Laboratory. That is, 1 to 2 g of a sample is prepared in said balance, and dried at 130°C until the weighing value becomes equilibrium (approximately for 15 minutes). The same sample is weighed 3 times and the average of the three values is determined as the water content.

The weight average molecular weight of a PHA was determined using Shimadzu's gel chromatography system (RI detection) equipped with Shodex K806L (300 x 8 mm, 2 columns-connected) (product of Showa Denko K.K.) with chloroform as a mobile phase. As the molecular weight standard sample, commercially available standard polystyrene was used. Moreover, the PHBH purity was determined by gas chromatography after methyl esterification of PHBH.

### (Comparative Example 1)

PHBH was produced by culturing Ralstonia eutropha which is internationally deposited on August 7, 1997 as Alcaligenes eutrophus AC32 (deposition number FERM BP-6038) obtained by introducing a PHA synthase group gene derived from Aeromonas caviae according to the method described in Example 1 of Japanese Kokai Publication 2001-340078.

After completion of the culture, the cell broth was sterilized at 60°C for 20 minutes. PHBH at the completion of the culture had the weight average molecular weight of 1,000,000, and a 3-hydroxyhexanoate (hereinafter referred to briefly as "3HH") composition of 6.2 mol%. The cultured broth was subjected to spray drying using a spray dryer "Pulvis GB22" manufactured by Yamato Scientific Co., Ltd. under the conditions of the heat current inlet temperature of 150°C, and the heat current outlet temperature of 60°C. The molecular weight of PHBH did not decrease at the time of spray drying under this condition. The water content of the obtained dried cell was 8.2% by weight.

10 g of this dried cell was added to 92 g of toluene heated to 90°C (PHBH 6% by weight), and an extraction was carried out for 1 hour. After completion of the extraction, the solution was transferred into a jacket-type pressurized filter kept hot at 90°C, and a PHBH extract was recovered by filtration. The recovered extract was kept hot at 90°C, and 30 g of n-heptane was gradually added while vigorously stirring the solution. After completion of the addition, the solution was gradually cooled to room temperature with vigorous stirring, and then white PHBH was precipitated. PHBH could be recovered easily by filtration, and the recovered PHBH was washed with methanol and dried in vacuum at 45°C after the washing. The recovery amount was 5.4 g (recovery rate 90%), and the purity was 98%. The weight average molecular weight was 390,000, i.e. as much as 61% decreased in 1 hour.

### (Example 1)

The dried cells obtained in Comparative Example 1 were dried in vacuum at 50°C for 5 hours. The water content of the obtained dried cells was 4.9% by weight. 10 g of the dried cells was subjected to a toluene extraction in the same manner as Comparative Example 1 to recover PHBH. The recovery amount was 5.5 g (recovery rate 92%), and the purity was 99%. The weight average molecular weight was 750,000, i.e. 25% decreased in 1 hour, but it was a sufficient molecular weight for processing.

### (Example 2)

The dried cells obtained in Comparative Example 1 were dried in vacuum at 50°C for 10 hours. The water content of the obtained dried cells was 2.6% by weight. 10 g of the dried cells was subjected to a toluene extraction in the same manner as Comparative Example 1 to recover PHBH. The recovery amount was 5.5 g (recovery rate 92%), and the purity was 99%. The weight average molecular weight was 800,000, i.e. only 20% decreased in 1 hour.

### (Example 3)

The dried cells obtained in Comparative Example 1 were dried in vacuum at 50°C for 20 hours. The water content of the obtained dried cells was 1.8% by weight. 10 g of the dried cells was subjected to a toluene extraction in the same manner as Comparative Example 1 to recover PHBH. The recovery amount was 5.5 g (recovery rate 92%), and the purity was 99%. The weight average molecular weight was 850,000, i.e. only 15% decreased in 1 hour.

From Examples 1 to 3, it was shown that the molecular weight decrease of PHA could be considerably prevented by decreasing the water content in dried cells.

### INDUSTRIAL APPLICABILITY

By the production method of the present invention, it becomes possible to produce and provide a biodegradable polyhydroxyalkanoate having a preferable processability on a commercial scale.

## Claims

1. A method for producing a polyhydroxyalkanoate
which comprises extracting a polyhydroxyalkanoate from a polyhydroxyalkanoate-containing biomass having the water content of 5% by weight or less using an extraction solvent, crystallizing, and recovering the resultant.

2. The method for producing a polyhydroxyalkanoate according to Claim 1,
wherein the water content in the biomass is controlled to 5% by weight or less by drying the biomass by heating.

3. The method for producing a polyhydroxyalkanoate according to Claim 2,
wherein the drying by heating of the biomass is carried out at 40°C or higher.

4. The method for producing a polyhydroxyalkanoate according to Claim 1,
wherein the polyhydroxyalkanoate-containing biomass having the water content of 5% by weight or less is prepared by concentration and/or azeotropic dehydration under the coexistence of a solvent.

5. The method for producing a polyhydroxyalkanoate according to any one of Claims 1 to 4,
wherein the extraction of a polyhydroxyalkanoate is carried out in one step without removing impurities other than the polyhydroxyalkanoate in the biomass.

6. The method for producing a polyhydroxyalkanoate according to any one of Claims 1 to 5,
wherein a polyhydroxyalkanoate crystal is precipitated by dissolving a polyhydroxyalkanoate in an extraction solvent, keeping the solution warm at 50°C or higher, adding a poor solvent for crystallization thereto, and further cooling said solution to below 50°C.

7. The method for producing a polyhydroxyalkanoate according to any one of Claims 1 to 6,
wherein the extraction solvent is at least one species selected from the group consisting of monohydric alcohols having 1 to 10 carbon atoms, aromatic hydrocarbons having 6 to 10 carbon atoms, ketones having 4 to 7 carbon atoms, and fatty acid alkyl esters having 5 to 8 carbon atoms.

8. The method for producing a polyhydroxyalkanoate according to Claim 7,
wherein the monohydric alcohols having 1 to 10 carbon atoms is at least one species selected from the group consisting of butanol, pentanol, hexanol, cyclohexanol, 1-methylcyclohexanol, 2-ethylhexanol, benzyl alcohol, heptanol, octanol, nonanol, decanol, and isomers thereof.

9. The method for producing a polyhydroxyalkanoate according to Claim 7,
wherein the aromatic hydrocarbons having 6 to 10 carbon atoms are at least one species selected from the group consisting of benzene, toluene, xylene, ethyl benzene, dimethoxy benzene, trimethyl benzene, cumene, butyl benzene, cymene, and isomers thereof.

10. The method for producing a polyhydroxyalkanoate according to Claim 7,
wherein the ketones having 4 to 7 carbon atoms are at least one species selected from the group consisting of methyl ethyl ketone, methyl butyl ketone, pentanon, hexanon, cyclohexanone, heptanone, and isomers thereof.

11. The method for producing a polyhydroxyalkanoate according to Claim 7,
wherein the fatty acid alkyl esters having 5 to 8 carbon atoms are at least one species selected from the group consisting of propyl acetate, butyl acetate, pentyl acetate, hexyl acetate, and isomers thereof.

12. The method for producing a polyhydroxyalkanoate according to any one of Claims 6 to 11,
wherein the poor solvent for crystallization is an aliphatic hydrocarbon having 6 to 12 carbon atoms with the boiling point of 60°C or higher.

13. The method for producing a polyhydroxyalkanoate according to Claim 12,
wherein the poor solvent for crystallization is at least one species selected from the group consisting of hexane, heptane, methylcyclohexane, octane, nonane, decane, dodecane, undecane, and isomers thereof.

14. The method for producing a polyhydroxyalkanoate according to any one of Claims 1 to 13,
wherein the weight ratio of the polyhydroxyalkanoate relative to the total amount of the polyhydroxyalkanoate and the extraction solvent in extracting the polyhydroxyalkanoate is within the range of 1 to 20% by weight.

15. The method for producing a polyhydroxyalkanoate according to any one of Claims 6 to 14,
wherein the poor solvent for crystallization is added such an amount that the weight ratio of the poor solvent for crystallization relative to the total amount of the poor solvent for crystallization and extraction solvent becomes 10 to 70% by weight.

16. The method for producing a polyhydroxyalkanoate according to any one of Claims 6 to 15,
wherein the extraction solvent is toluene and the poor solvent for crystallization is heptane.

17. The method for producing a polyhydroxyalkanoate according to any one of Claims 1 to 16,
wherein the polyhydroxyalkanoate is a copolymer obtainable by copolymerizing at least two species of monomers selected from the group consisting of 3-hydroxybutyrate, 3-hydroxyvalerate, 3-hydroxypropionate, 4-hydroxybutyrate, 4-hydroxyvalerate, 5-hydroxyvalerate, 3-hydroxyhexanoate, 3-hydroxyheptanoate, 3-hydroxyoctanoate, 3-hydroxynonanoate and 3-hydroxydecanoate.

18. The method for producing a polyhydroxyalkanoate according to any one of Claims 1 to 17,
wherein the polyhydroxyalkanoate is a copolymer composed of 3-hydroxyhexanoate and at least one species of hydroxyalkanoates other than 3-hydroxyhexanoate.

19. The method for producing a polyhydroxyalkanoate according to any one of Claims 1 to 18,
wherein the polyhydroxyalkanoate is a copolymer composed of 3-hydroxyhexanoate and 3-hydroxybutyrate.

20. The method for producing a polyhydroxyalkanoate according to any one of Claims 1 to 19,
wherein the biomass is a microorganism.

21. The method for producing a polyhydroxyalkanoate according to any one of Claims 1 to 20,
wherein the polyhydroxyalkanoate is produced by at least one species of cell selected from the group consisting of species belonging to the genus Aeromonas, Alcaligenes, Azotobacter, Bacillus, Clostridium, Halobacterium, Nocardia, Rhodospirillum, Pseudomonas, Ralstonia, Zoogloea, Candida, Yarrowia, and Saccharomyces.

22. The method for producing a polyhydroxyalkanoate according to any one of Claims 1 to 21,
wherein the polyhydroxyalkanoate-containing biomass is a transformant obtainable by introducing a polyhydroxyalkanoate synthetic gene group derived from Aeromonas caviae.

23. The method for producing a polyhydroxyalkanoate according to Claim 22,
wherein the polyhydroxyalkanoate-containing biomass is Ralstonia eutropha obtainable by introducing a polyhydroxyalkanoate synthetic gene group derived from Aeromonas caviae.
